# EUROPÄISCHE PATENTSCHRIFT

(11) **EP 2 547 379 B1**
(45) Veröffentlichungstag und Bekanntmachung des Hinweises auf die Patenterteilung: **27.02.2019**
(21) Anmeldenummer: 11709646.1
(22) Anmeldetag: 18.03.2011
(51) Int. Cl.: A61M 1/16, B01J 20/00, A61M 1/28, A61K 31/74, B01J 20/26, A61K 45/06

(54) **MOLEKULAR GEPRÄGTE POLYMERE FÜR DIE ELIMINIERUNG VON METABOLITEN**
MOLECULARLY IMPRINTED POLYMERS FOR ELIMINATING METABOLITES
POLYMÈRES À EMPREINTE MOLÉCULAIRE POUR L'ÉLIMINATION DE MÉTABOLITES

(30) Priorität: 19.03.2010 DE 102010012199
(43) Veröffentlichungstag der Anmeldung: 23.01.2013
(73) Patentinhaber: Fresenius Medical Care Deutschland GmbH, 61352 Bad Homburg (DE)
(72) Erfinder: FICHERT, Thomas, 48231 Warendorf (DE); BICHLMAIER, Ingo, 61206 Wöllstadt (DE)
(74) Vertreter: Breuninger, Marcus
(86) Internationale Anmeldenummer: PCT/EP2011/001357
(87) Internationale Veröffentlichungsnummer: WO 2011/113607

(56) Entgegenhaltungen:
- WO-A1-2009/104323
- WO-A2-2010/081076
- JP-A- 2005 264 013
- US-A1- 2008 200 434
- US-A1- 2008 241 185
- HSIEH R-Y ET AL: "Designing a molecularly imprinted polymer as an artificial receptor for the specific recognition of creatinine in serums", BIOMATERIALS, ELSEVIER SCIENCE PUBLISHERS BV., BARKING, GB, Bd. 27, Nr. 9, 1. März 2006 (2006-03-01), Seiten 2083-2089, XP025097659, ISSN: 0142-9612, DOI: DOI:10.1016/J.BIOMATERIALS.2005.09.024 [gefunden am 2006-03-01] in der Anmeldung erwähnt
- TSAI H A ET AL: "Synthesis of creatinine-imprinted poly(beta-cyclodextrin) for the specific binding of creatinine", BIOMATERIALS, ELSEVIER SCIENCE PUBLISHERS BV., BARKING, GB, Bd. 26, Nr. 15, 1. Mai 2005 (2005-05-01), Seiten 2759-2766, XP025280699, ISSN: 0142-9612, DOI: DOI:10.1016/J.BIOMATERIALS.2004.07.037 [gefunden am 2005-05-01]
- LIU Z ET AL: "Absorption performance of iodixanol-imprinted polymers in aqueous and blood plasma media", ACTA BIOMATERIALIA, ELSEVIER, AMSTERDAM, NL, Bd. 6, Nr. 6, 1. November 2009 (2009-11-01), Seiten 2003-2012, XP027035715, ISSN: 1742-7061 [gefunden am 2009-11-16]
- RAYMOND VANHOLDER ET AL: "Review on uremic toxins: Classification, concentration, and interindividual variability", KIDNEY INTERNATIONAL, NATURE PUBLISHING GROUP, LONDON, GB, Bd. 63, Nr. 5, 1. Mai 2003 (2003-05-01), Seiten 1934-1943, XP009088717, ISSN: 0085-2538, DOI: DOI:10.1046/J.1523-1755.2003.00924.X in der Anmeldung erwähnt
- CHANIN NANTASENAMAT ET AL: "Quantitative prediction of imprinting factor of molecularly imprinted polymers by artificial neural network", JOURNAL OF COMPUTER-AIDED MOLECULAR DESIGN, KLUWER ACADEMIC PUBLISHERS, DO, Bd. 19, Nr. 7, 1. Juli 2005 (2005-07-01), Seiten 509-524, XP019248191, ISSN: 1573-4951
- Bulent Tokgoz: "Clinical advantages of peritoneal dialysis", Peritoneal Dialysis International, 16 May 2009 (2009-05-16), pages S59-S61, XP055088438, Retrieved from the Internet: URL:http://www.pdiconnect.com/content/29/S upplement_2/S59.full.pdf#page=1&view=FitH [retrieved on 2013-11-14]

## Beschreibung

Die vorliegende Erfindung betrifft durch Retentionssolute geprägte Polymere, deren Verwendung sowie Zusammensetzungen, die diese enthalten.

Bei der Dialysebehandlung, insbesondere bei der Peritonealdialysebehandlung, werden Osmotika eingesetzt, die dazu dienen, dem Dialysepatienten überschüssiges Wasser zu entziehen.

Das Peritonealdialyseverfahren beruht darauf, dass eine Lösung, die osmotisch aktive Verbindungen enthält, über einen Katheter in die Bauchhöhle des Dialysepatienten eingebracht wird. Diese Lösung wird für eine bestimmte Zeit (üblicherweise einige Stunden) in der Bauchhöhle des Patienten belassen und entfaltet dort ihre osmotische Wirkung; d.h. dem Patienten wird körpereigenes Wasser in die Bauchhöhle entzogen. Nach einer bestimmten Verweildauer wird die nunmehr verdünnte Peritonealdialyselösung über einen Katheter abgelassen.

Dieses Prinzip findet in verschiedenen Verfahren der Peritonealdialysebehandlung Anwendung. Nach Bedarf können beispielsweise die Verfahren der intermittierenden (IPD), nächtlichen intermittierenden (NIPD), kontinuierlichen zyklischen (CCPD) oder kontinuierlichen ambulanten Peritonealdialyse (CAPD), angewendet werden. Bei IPD, NIPD und CCPD kommen Geräte zum Einsatz, die den Patienten bei der Durchführung des Peritonealdialyseverfahrens unterstützen. Die CAPD ist ein manuelles Verfahren.

Durch die Zugabe von osmotisch aktiven Verbindungen soll insbesondere gewährleistet werden, dass der osmotische Druck der Peritonealdialyselösung während der gesamten Verweildauer in der Bauchhöhle hoch genug ist, um dem Patienten Wasser zu entziehen; d.h. Wasser geht aus dem Kreislauf des Patienten in die Bauchhöhle über (Ultrafiltration).

Eine wesentliche Aufgabe der Nieren ist neben der Wasserausscheidung auch die Eliminierung von Stoffwechselprodukten (renale Eliminierung).

Bei Patienten mit eingeschränkter Nierenfunktion ist die Ausscheidung von Metaboliten, die ausschließlich oder überwiegend renal eliminiert werden, jedoch gestört, so dass es zu einer Retention (Rückhaltung) dieser gelösten oder eiweißgebundenen Stoffwechselprodukte im Körper des Patienten kommt. Diese nicht-eliminierten Stoffwechselprodukte werden auch als urämische Retentionssolute ("uremic retention solutes") bezeichnet (vgl. Vanholder et al., Kidney International, 63 (2003), 1934-1943).

Es ist bekannt, dass diese urämischen Retentionssolute zu pathophysiologischen Erscheinungen im nierenisuffizienten Patienten führen können (urämisches Syndrom; vgl. Vanholder et al., Hemodialysis International, 7 (2003), 156-161).

Die urämischen Retentionssolute, die zu pathophysiologischen Erscheinungen führen, werden auch als urämische Toxine bezeichnet. Für die Gesundheit des Dialysepatienten ist es wichtig, dass die urämischen Retentionssolute aus dem Körper eliminiert werden.

Bei der Dialysebehandlung wird dem Dialysepatienten überschüssiges Körperwasser entzogen. Auch Metabolite werden hierbei entlang des Konzentrationsgefälles nichtselektiv aus dem Körperwasser des Patienten in die Dialyselösung eliminiert. Jedoch kann diese Eliminierung in ungenügender Menge stattfinden. Somit kann es auch in Dialysepatienten zu einer Retention an Metaboliten kommen, die wiederum zum urämischen Syndrom beitragen können.

In der Literatur ist die Synthese molekular geprägter Polymere, die Kreatinin binden, beschrieben (Tsai und Syu, Biomaterials, 26 (2005), 2759-2766).

WO 2009/104323 A1 und US 2011/003937 offenbaren molekular geprägte Polymere, welche durch wenigstens ein urämisches Toxin/Retentionssolut geprägt sind sowie deren Verwendung in der Dialysebehandlung.

US 2008/241185 A1 beschreibt molekular geprägte Polymere und deren Verwendung in der Dialysebehandlung.

Hsieh et al., (Biomaterials, 27 (2006), 2083-2089) offenbaren molekular geprägte Polymere bestehend aus Cyclodextrinmonomeren, die mit Kreatinin als Mustermolekül/Template molekular geprägt wurden.

WO 2010/081076 betrifft molekular geprägte Polymernanopartikel und deren Verwendung in biomakromolekularen Reinigungsverfahren.

Aufgabe der vorliegenden Erfindung ist es die Eliminierung von urämischen Retentionssoluten während der Dialysebehandlung zu erhöhen.

Diese Aufgabe wird durch den Gegenstand der Patentansprüche gelöst.

Es wurde gefunden, dass der Einsatz von Polymeren, die durch urämische Retentionssolute geprägt sind, die Eliminierung der Metabolite bei der Dialysebehandlung erhöht. Dies ist eine Folge der erhöhten spezifischen Bindung des Retentionssoluts zum geprägten Polymer. Somit kommt es zu einer erhöhten Eliminierung des urämischen Retentionssoluts.

Zudem weisen diese molekular geprägten Polymere auch eine hohe osmotische Wirksamkeit auf, so dass sie als Osmotika in Dialyselösungen eingesetzt werden können.

Die erfindungsgemäßen molekular geprägten Polymere weisen also einen dualen Wirkmechanismus auf: zum einen binden sie bestimmte urämische Retentionssolute und erhöhen somit ihre Eliminierung (spezifische Eliminierung) bei der Dialysebehandlung, zum anderen weisen sie selbst osmotische Wirksamkeit auf und unterstützen somit den Wasserentzug und die damit verbundene nichtspezifische Eliminierung von Metaboliten entlang des Konzentrationsgradienten.

Ein erster Gegenstand dieser Erfindung betrifft ein molekular geprägtes Polymer, das durch wenigstens ein urämisches Retentionssolut geprägt ist, zur Verwendung in der Dialysebehandlung.

Im Sinne dieser Beschreibung steht der Begriff "molekular geprägtes Polymer" für ein Polymer, das durch Polymerisieren oder Quervernetzen von Monomeren in Gegenwart eines Mustermoleküls ("Template") hergestellt wird. Als Mustermolekül wird hierin wenigstens ein urämisches Retentionssolut verwendet.

Für die Zwecke dieser Beschreibung umfasst der Begriff "Monomer" eine Verbindung, welche polymerisiert und/oder quervernetzt werden kann (vgl. auch Pure and Applied Chemistry, 68 (1996), 2287). Monomere, die zur Polymerisierung eingesetzt werden können sind dem Fachmann bekannt.

Im Sinne dieser Beschreibung können Monomere Verbindungen sein, die selbst aus nur einer Einzelverbindung bestehen, jedoch umfasst der Begriff auch Verbindungen, die aus mehr als einer Einzelverbindung dimerisiert, oligomerisiert oder polymerisiert worden sind, wie beispielsweise Dimere, Oligomere oder Polymere.

Im Sinne dieser Beschreibung umfasst der Begriff Dimer eine Verbindung, die aus 2 Einzelverbindungen durch Dimerisierung hergestellt worden ist. Im Sinne dieser Beschreibung umfasst der Begriff Oligomer eine Verbindung, die aus 3 bis 9 Einzelverbindungen durch Polymerisation (Oligomerisation) hergestellt worden ist. Im Sinne dieser Beschreibung steht der Begriff Polymer für eine makromolekulare Verbindung, die aus ≥10 Einzelverbindungen durch Polymerisation hergestellt worden ist.

In einer bevorzugten Ausführungsform ist das erfindungsgemäße molekular geprägte Polymer geprägt durch wenigstens ein urämisches Retentionssolut, das ausgewählt ist aus der Gruppe 1-Methyladenosin, 1-Methylguanosin, 1-Methylinosin, asymmetrisches Dimethylarginin, α-Keto-δ-guanidinovaleriansäure, α-N-Acetylarginin, Arab(in)itol, Argininsäure, Benzylalkohol, β-Guanidinopropionsäure, β-Lipotropin, Kreatin, Kreatinin, Cytidin, Dimethylglycin, Erythritol, γ-Guanidino buttersäure, Guanidin, Guanidinoessigsäure, Guanidonobernsteinsäure, Hypoxanthin, Malondial-dehyd, Mannitol, Methylguanidin, Myoinositol, N²,N²-Dimethylguanosin, N⁴-Acetylcytidin, N⁶-Methyladenosin, N⁶-Threonylcarbamoyladenosin, Orotsäure, Orotidin, Oxalat, Phenylacetylglutamin, Pseudouridin, symmterisches Dimethylarginin, Sorbitol, Taurocyamin, Threitol, Thymin, Uracil, Harnstoff, Harnsäure, Uridin, Xanthin, Xanthosin, 2-Methoxyresorcinol, 3-Deoxyglucoson, 3-Carboxy-4-methyl-5-propyl-2-furanproprionsäure, Fructoselysin, Glyoxal, Hippursäure, Homocystein, Hydroquinon, Indol-3-essigsäure, Indoxylsulfat, Kinurenin, Kynurensäure, Leptin, Melatonin, Methylglyoxal, N^{ε}-(carboxymethyl)lysin, p-Cresol, Pentosidin, Phenol, p-Hydroxyhippursäure, Putrescin, Quinolinsäure, Retinol-bindendes Protein, Spermidin, Spermin, Adrenomedullin, atriales natriuretisches Peptid, β₂-microglobulin, β-endorphin, Cholecystokinin, Clara Cell Protein (CC16), Komplementfaktor D, Cystatin C, Degranulation Inhibiting Protein, Delta-sleep Inducing Peptid, Endothelin, Hyaluronsäure, Interleukin-1β, Interleukin-6, κ-Ig Light Chain, λ-Ig Light Chain, Leptin, Methionin-Enkephalin, Neuropeptid Y, Parathyroidhormon, Retinol-bindendes Protein, Tumornekrosefaktor-α, 1-alkyl-2-formyl-3,4-glycosyl-pyrrol, 2-(2-Fuoryl)-4(5)-(2-furanyl)-1H-imidazole, 3-Deoxyfructoson, 3-Hydroxykinurenin, 4-Hydroxynonenal, AOPP (Advanced Oxidation Protein Products), Advanced Glycation End Products-β₂-Microglobulin, Anthranilsäure, β₂-Microglobulinfragmente, Cadaverin, Crossline, Dimethylamin, Guanosin, Imidazolon, Malonaldehyd, Malondialdehyd, Methylamin, N^{ε}-carboxyethyllysine, Organische Chloramine, oxidiertes Low-Density-Lipoprotein (oxLDL), Parathyroidhormonfragmente, Pyrralin, Pyrrolaldehyd, und Trimethylamin.

Das mittlere Molekulargewicht des erfindungsgemäßen Polymers beträgt vorzugsweise 2000 bis 30000 g/mol, bevorzugter 2500 bis 26000 g/mol, noch bevorzugter 3000 bis 22000 g/mol, noch bevorzugter 3500 bis 20000 g/mol, am Bevorzugtesten 4000 bis 18000 g/mol und insbesondere 5000 bis 15000 g/mol.

In einer weiteren bevorzugten Ausführungsform beträgt das mittlere Molekulargewicht des erfindungsgemäßen Polymers 15000 bis 25000 g/mol, insbesondere 18000 bis 22000 g/mol.

Das erfindungsgemäße Polymer weist vorzugsweise einen durchschnittlichen Polymerisationsgrad von 10 bis 170, bevorzugter von 11 bis 130, noch bevorzugter von 12 bis 100, am Bevorzugtesten von 13 bis 80 und insbesondere von 14 bis 50 auf.

In einer weiteren bevorzugten Ausführungsform ist der durchschnittliche Polymerisationsgrad des erfindungsgemäßen molekular geprägten Polymers 80 bis 140, bevorzugter 85 bis 135, noch bevorzugter 90 bis 130, am Bevorzugtesten 95 bis 125 und insbesondere 100 bis 120.

Vorzugsweise weist eine 7,5 gewichtsprozentige wässrige Lösung des erfindungsgemäßen molekular geprägten Polymers eine theoretische Osmolarität von >5 mosm/L, bevorzugter von ≥7,5 mosm/L, noch bevorzugter von ≥10,0 mosm/L, am Bevorzugtesten von ≥12,5 mosm/L und insbesondere von ≥15 mosm/L auf.

Zum Zwecke dieser Beschreibung steht der Ausdruck "theoretische Osmolarität" für die theoretisch berechnete Osmolarität. Methoden zur Berechnung dieses Werts sind dem Fachmann bekannt.

In einer bevorzugten Ausführungsform beträgt der kolloidosmotische Druck einer 7,5 gewichtsprozentigen Lösung des erfindungsgemäßen molekular geprägten Polymers ≥50 mosm/L oder ≥60 mosm/L, bevorzugter ≥70 mosm/L oder ≥80 mosm/L, noch bevorzugter ≥90 mosm/L oder ≥100 mosm/L, am Bevorzugtesten ≥110 mosm/L oder ≥120 mosm/L und insbesondere ≥130 mosm/L oder ≥140 mosm/L.

In einer weiteren bevorzugten Ausführungsform beträgt der kolloidosmotische Druck einer 7,5 gewichtsprozentigen Lösung des erfindungsgemäßen Polymers ≥150 mosm/L oder ≥160 mosm/L, bevorzugter ≥170 mosm/L oder ≥180 mosm/L, noch bevorzugter ≥190 mosm/L oder ≥200 mosm/L, am Bevorzugtesten ≥210 mosm/L oder ≥220 mosm/L und insbesondere ≥230 mosm/L oder ≥240 mosm/L.

In einer weiteren bevorzugten Ausführungsform beträgt der kolloidosmotische Druck einer 7,5 gewichtsprozentigen Lösung des erfindungsgemäßen Polymers 50 bis 500 mosm/L, bevorzugter 75 mosm/L bis 400 mosm/L, noch bevorzugter 100 bis 300 mosm/L, am Bevorzugtesten 110 mosm/L bis 275 mosm/L und insbesondere 120 mosm/L bis 250 mosm/L.

In einer weiteren bevorzugten Ausführungsform beträgt der kolloidosmotische Druck einer 7,5 gewichtsprozentigen Lösung des erfindungsgemäßen Polymers 100 bis 500 mosm/L, bevorzugter 100 mosm/L bis 400 mosm/L, noch bevorzugter 100 bis 350 mosm/L, am Bevorzugtesten 100 mosm/L bis 325 mosm/L und insbesondere 100 mosm/L bis 290 mosm/L.

Zum Zwecke dieser Beschreibung steht der Ausdruck "kolloidosmotischer Druck" für den experimentell gemessenen osmotischen Druck der Lösung, der sich aus dem osmotischen und onkotischen Druck zusammensetzt. Geeignete Methoden zur experimentellen Bestimmung dieses Werts sind dem Fachmann bekannt.

Die Osmolalität einer 7,5 gewichtsprozentigen wässrigen Lösung des erfindungsgemäßen Polymers beträgt vorzugsweise ≥5 mosm/kg, bevorzugter ≥7,5 mosm/kg, noch bevorzugter ≥10 mosm/kg, am Bevorzugtesten ≥12,5 mosm/kg und insbesondere ≥15 mosm/kg.

Zum Zwecke dieser Beschreibung steht der Begriff "Osmolalität" für die mittels Gefrierpunktserniedrigung experimentell bestimmten Osmolalität der Lösung. Methoden zur Bestimmung der Gefrierpunktserniedrigung sind dem Fachmann bekannt.

Die Wasserlöslichkeit des erfindungsgemäßen molekular geprägten Polymers beträgt vorzugsweise ≥5 g/L, bevorzugter ≥10 g/L, noch bevorzugter ≥20 g/L, am Bevorzugtesten ≥50 g/l und insbesondere ≥75 g/L.

Das erfindungsgemäße molekular geprägte Polymer kann selbst mit verschiedenen Resten substituiert sein. Vorzugsweise ist das erfindungsgemäße Polymer mit kationischen, anionischen, deprotonierbaren oder protonierbaren Resten substituiert, die die osmotische Wirksamkeit des Polymers erhöhen. Bevorzugte deprotonierbare bzw. anionische Reste sind beispielsweise abgeleitet von Dicarbonsäuren oder Tricarbonsäuren.

Geeignete Dicarbonsäuren sind beispielsweise ausgewählt aus der Gruppe Oxalsäure, Oxalessigsäure, Ketoglutarsäure, Glutaminsäure, Asparaginsäure, Fumarsäure, Maleinsäure, Äpfelsäure, Malonsäure, Bernsteinsäure, Glutarsäure, Adipinsäure, Pimelinsäure, Suberinsäure, Azelainsäure, Sebacinsäure. In einer weiteren bevorzugten Ausführungsform ist die Dicarbonsäure Oxalsäure, Glutaminsäure, Asparaginsäure, Maleinsäure oder Bernsteinsäure. Insbesondere bevorzugt sind Maleinsäure und Bernsteinsäure.

Geeignete Tricarbonsäuren sind vorzugsweise Citronensäure oder Isocitronensäure, insbesondere Citronensäure.

Das erfindungsgemäße Polymer kann mit Dicarbonsäuren und/oder Tricarbonsäuren verestert sein. Das erfindungsgemäße Polymer weist dabei einen Substitutionsgrad von 0,01 bis 3, bevorzugter von 0,05 bis 2,5, noch bevorzugter von 0,1 bis 2, am Bevorzugtesten von 0,25 bis 1,5 und insbesondere von 0,5 bis 1 auf.

In einer weiteren bevorzugten Ausführungsform weist das erfindungsgemäße Polymer einen Substitutionsgrad von 0,02±0,01 oder 0,05±0,025, bevorzugter von 0,1±0,05, noch bevorzugter von 0,5±0,25, am Bevorzugtesten von 1±0,5 und insbesondere von 1,5±0,75 auf.

In einer besonders bevorzugten Ausführungsform weist das erfindungsgemäße Polymer einen Substitutionsgrad von 0,02±0,005 oder 0,05±0,0125, bevorzugter von 0,1±0,025, noch bevorzugter von 0,5±0,125, am Bevorzugtesten von 1±0,25 und insbesondere von 1,5±0,375 auf.

Das molekular geprägte Polymer ist vorzugsweise abgeleitet von a) wenigstens einem Monomer oder von b) wenigstens einem Monomer und wenigstens einem Vernetzungsmittel.

Das Monomer ist vorzugsweise ausgewählt aus der Gruppe der Saccharide, der Aminosäuren, Peptide oder olefinischen Monomere.

Bevorzugte Saccharide sind Monomere wie Glucose, Fructose, Arabinose, Xylose, Galactose, Mannose, N-Acetylglucosamin oder Glucosamin sowie Dimere, Oligomere oder Polymere, die aus den genannten Saccharid-Monomeren aufgebaut sind. Insbesondere bevorzugt sind die Monomere Glucose und Fructose sowie Dimere, Oligomere und Polymere, die aus Glucose und/oder Fructose aufgebaut sind. Die Oligosaccharide können auch cyclisch sein, wie beispielsweise α-, β- oder γ-Cyclodextrin. Bevorzugte Polymere sind Inulin oder Stärke sowie deren Derivate, vorzugsweise abgebaute Stärke (Stärkehydrolysate; hydrolysiertes Polydextrin).

Bevorzugte Aminosäuren sind ausgewählt aus der Gruppe Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin. Bevorzugte Peptide sind Dimere, Oligomere oder Polymere, die aus Aminosäuren aufgebaut sind, die aus der vorstehend genannten Gruppe ausgewählt sind.

Als olefinische Monomere kommt vorzugsweise jede beliebige Verbindung in Betracht, die mindestens eine C-C-Doppelbindung enthält, die einer Polymerisierung zugänglich ist. Solche olefinischen Monomere sind dem Fachmann bekannt. Bevorzugte olefinische Monomere sind 4-Vinylpyridin und Divinylbenzen.

Das Vernetzungsmittel ist vorzugsweise ausgewählt aus der Gruppe Glyoxal, 1,2-Dihalogenethan, 1,3-Dihalogenpropan, Halogencarbonsäurehalogenid, Epichlorhydrin, 4-Chlor-1,2-epoxybutan, 1,2,3,4-Diepoxybutan, Tetramethylendiisocyanat und Hexamethylendiisocyanat.

Das erfindungsgemäße Polymer eignet sich auch als Osmotikum zur Einstellung der Tonizität von Arzneimitteln, insbesondere von Arzneilösungen zur parenteralen Applikation.

In einer bevorzugten Ausführungsform findet das erfindungsgemäße Polymer Verwendung in der Dialysebehandlung, vorzugsweise in der Hämo- und/oder Peritonealdialysebehandlung.

Das erfindungsgemäße Polymer ist insbesondere zur Verwendung in der Peritonealdialysebehandlung geeignet.

Ein weiterer Gegenstand dieser Erfindung betrifft ein molekular geprägtes Polymer erhältlich durch ein Verfahren umfassend die Schritte:
a) Mischen wenigstens eines Monomers mit wenigstens einem urämischen Retentionssolut;
b) Polymerisieren und/oder Quervernetzen der Monomere;
zur Verwendung in der Dialysebehandlung.

Die Monomere, urämischen Retentionssolute und Vernetzungsmittel sind wie oben stehend definiert.

Ein weiterer Gegenstand dieser Erfindung betrifft ein Verfahren zur Herstellung des erfindungsgemäßen molekular geprägten Polymers, umfassend die Schritte
a. Mischen wenigstens eines Monomers mit wenigstens einem urämischen Retentionssolut ausgewählt aus der Gruppe 1-Methyladenosin, 1-Methylguanosin, 1-Methylinosin, asymmetrisches Dimethylarginin, α-Keto-δ-guanidinovaleriansäure, α-N-Acetylarginin, Arab(in)itol, Argininsäure, Benzylalkohol, β-Guanidinopropionsäure, β-Lipotropin, Kreatin, Cytidin, Dimethylglycin, Erythritol, γ-Guanidinobuttersäure, Guanidin, Guanidinoessigsäure, Guanidonobernsteinsäure, Hypoxanthin, Malondialdehyd, Mannitol, Methylguanidin, Myoinositol, N²,N²-Dimethylguanosin, N⁴-Acetylcytidin, N⁶-Methyladenosin, N⁶-Threonylcarbamoyladenosin, Orotsäure, Orotidin, Oxalat, Phenylacetylglutamin, Pseudouridin, symmterisches Dimethylarginin, Sorbitol, Taurocyamin, Threitol, Thymin, Uracil, Harnsäure, Uridin, Xanthin, Xanthosin, 2-Methoxyresorcinol, 3-Deoxyglucoson, 3-Carboxy-4-methyl-5-propyl-2-furanproprionsäure, Fructoselysin, Glyoxal, Hippursäure, Homocystein, Hydroquinon, Indol-3-essigsäure, Indoxylsulfat, Kinurenin, Kynurensäure, Leptin, Melatonin, Methylglyoxal, N^{ε}-(carboxymethyl)lysin, p-Cresol, Pentosidin, Phenol, p-Hydroxyhippursäure, Putrescin, Quinolinsäure, Retinol-bindendes Protein, Spermidin, Spermin, Adrenomedullin, atriales natriuretisches Peptid, β₂-microglobulin, β-endorphin, Cholecystokinin, Clara Cell Protein (CC16), Komplementfaktor D, Cystatin C, Degranulation Inhibiting Protein, Delta-sleep Inducing Peptid, Endothelin, Hyaluronsäure, Interleukin-1β, Interleukin-6, κ-Ig Light Chain, λ-Ig Light Chain, Leptin, Methionin-Enkephalin, Neuropeptid Y, Parathyroidhormon, Retinol-bindendes Protein, Tumornekrosefaktor-α, 1-Alkyl-2-formyl-3,4-glycosyl-pyrrol, 2-(2-Fuoryl)-4(5)-(2-furanyl)-1H-imidazole, 3-Deoxyfructoson, 3-Hydroxykinurenin, 4-Hydroxynonenal, AOPP (Advanced Oxidation Protein Products), Advanced Glycation End Products-β₂-Microglobulin, Anthranilsäure, β₂-Microglobulinfragmente, Cadaverin, Crossline, Dimethylamin, Guanosin, Imidazolon, Malonaldehyd, Malondialdehyd, Methylamin, N^{ε}-carboxyethyllysine, Organische Chloramine, oxidiertes Low-Density-Lipoprotein (oxLDL), Parathyroidhormonfragmente, Pyrralin, Pyrrolaldehyd, und Trimethylamin; und
b. Starten der Polymerisations- und/oder Quervernetzungsreaktion.

Die Polymerisationsreaktion kann bereits während des Mischens der Komponenten beginnen oder durch Zugabe geeigneter Polymerisationsstarter wie beispielsweise AIBN gestartet werden. Polymerisationsstarter sind dem Fachmann bekannt.

Beispielsweise ist es auch möglich die Polymerisation durch Erhöhung der Temperatur und/oder das Einwirken von elektromagnetischer Strahlung zu starten.

Die Quervernetzungsreaktion wird vorzugsweise durch Zugabe eines Vernetzungsmittels gestartet. Geeignete Vernetzungsmittel sind wie oben stehend definiert.

Ein weiterer Gegenstand dieser Erfindung betrifft Dialyselösungen enthaltend wenigstens ein erfindungsgemäßes molekular geprägtes Polymer.

In einer bevorzugten Ausführungsform ist die erfindungsgemäße Dialyselösung eine Hämodialyselösung oder eine Peritonealdialyselösung. Die erfindungsgemäße Dialyselösung ist insbesondere eine Peritonealdialyselösung.

Darreichungsformen, die in der Dialysebehandlung eingesetzt werden, sind vorzugsweise Konzentrate in Mehrkomponenten-Systemen oder gebrauchsfertige Dialyselösungen.

Für die Zwecke dieser Erfindung umfasst der Ausdruck "Dialyselösung" eine gebrauchsfertige Darreichungsform zur Dialysebehandlung, d.h. eine flüssige Zubereitung, die als solche zur Applikation geeignet ist. Insbesondere muss die Dialyselösung vor der Applikation nicht verdünnt und/ oder mit anderen Zubereitungen gemischt werden.

Im Gegensatz zu den vorstehend beschriebenen Dialyselösungen werden Konzentrate, die entweder in flüssiger, halbfester oder fester Form vorliegen können, vor der Applikation mit Wasser oder wässrigen Lösungen verdünnt oder in Wasser oder wässrigen Lösungen gelöst. In analoger Weise müssen die Komponenten eines Mehrkomponenten-Systems vor der Applikation miteinander gemischt werden um eine gebrauchsfertige Dialyselösung zu erhalten. Konzentrate und Mehrkomponenten-Systeme können also als Vorstufe der erfindungsgemäßen Dialyselösung angesehen werden.

Die erfindungsgemäße Dialyselösung ist vorzugsweise eine Hämodialyse- oder eine Peritonealdialyselösung. Hämodialyse- und Peritonealdialyselösungen enthalten üblicherweise Elektrolyte in einer Konzentration, die im Wesentlichen der Plasma-Elektrolyt-Konzentration entspricht. Elektrolyte umfassen üblicherweise Natrium-, Kalium-, Calcium-, Magnesium- und Chlorid-Ionen.

Dialyselösungen haben üblicherweise einen physiologisch verträglichen pH-Wert. Dies wird vorzugsweise erreicht durch Puffer (Puffer-Systeme), die selbst zum Gesamtgehalt an Elektrolyten beitragen können. Die Puffer sind vorzugsweise Hydrogencarbonat, Lactat oder Pyruvat.

Ferner besitzen Dialyselösungen üblicherweise eine physiologisch verträgliche Osmolarität. Dies wird in der Regel erreicht durch die in der Dialyselösung enthaltenen Elektrolyte und erfindungsgemäßen molekular geprägten Polymere, die als osmotisch aktive Verbindungen (Osmotika) in der gewünschten Konzentration physiologisch verträglich sind.

Die erfindungsgemäße Dialyselösung besitzt eine Osmolarität im Bereich von vorzugsweise 200 bis 550 mosm/L.

Im Fall, dass die erfindungsgemäße Dialyselösung eine Hämodialyselösung ist, beträgt die Osmolarität vorzugsweise 200 bis 350 mosm/L oder 210 bis 340 mosm/L, bevorzugter 220 bis 330 mosm/L, noch bevorzugter 230 bis 320 mosm/L, am Bevorzugtesten 240 bis 310 mosm/L und insbesondere 250 bis 300 mosm/L. Verfahren zur Messung der Osmolarität und des osmotischen Drucks sind dem Fachmann bekannt. Beispielsweise können diese mit Hilfe eines Membranosmometers oder anderen geeigneten Messmethoden bestimmt werden.

Im Fall, dass die erfindungsgemäße Dialyselösung eine Peritonealdialyselösung ist, beträgt die Osmolarität vorzugsweise 200 bis 570 mosm/L oder 210 bis 560 mosm/L, bevorzugter 220 bis 550 mosm/L, noch bevorzugter 230 bis 540 mosm/L, am Bevorzugtesten 240 bis 530 mosm/L und insbesondere 250 bis 520 mosm/L. In einer bevorzugten Ausführungsform beträgt die Osmolarität 250 ± 50 mosm/L oder 250 ± 45 mosm/L, bevorzugter 250 ± 35 mosm/L, noch bevorzugter 250 ± 25 mosm/L, am Bevorzugtesten 250 ± 15 mosm/L und insbesondere 250 ± 10 mosm/L. In einer weiteren bevorzugten Ausführungsform beträgt die Osmolarität 300 ± 50 mosm/L oder 300 ± 45 mosm/L, bevorzugter 300 ± 35 mosm/L, noch bevorzugter 300 ± 25 mosm/L, am Bevorzugtesten 300 ± 15 mosm/L und insbesondere 300 ± 10 mosm/L. In einer weiteren bevorzugten Ausführungsform beträgt die Osmolarität 350 ± 50 mosm/L oder 350 ± 45 mosm/L, bevorzugter 350 ± 35 mosm/L, noch bevorzugter 350 ± 25 mosm/L, am Bevorzugtesten 350 ± 15 mosm/L und insbesondere 300 ± 10 mosm/L. In einer weiteren bevorzugten Ausführungsform beträgt die Osmolarität 400 ± 50 mosm/L oder 400 ± 45 mosm/L, bevorzugter 400 ± 35 mosm/L, noch bevorzugter 400 ± 25 mosm/L, am Bevorzugtesten 400 ± 15 mosm/L und insbesondere 300 ± 10 mosm/L. In einer weiteren bevorzugten Ausführungsform beträgt die Osmolarität 450 ± 50 mosm/L oder 450 ± 45 mosm/L, bevorzugter 450 ± 35 mosm/L, noch bevorzugter 450 ± 25 mosm/L, am Bevorzugtesten 450 ± 15 mosm/L und insbesondere 450 ± 10 mosm/L. In einer weiteren bevorzugten Ausführungsform beträgt die Osmolarität 500 ± 50 mosm/L oder 500 ± 45 mosm/L, bevorzugter 500 ± 35 mosm/L, noch bevorzugter 500 ± 25 mosm/L, am Bevorzugtesten 500 ± 15 mosm/L und insbesondere 500 ± 10 mosm/L.

Die erfindungsgemäße Dialyselösung hat einen pH-Wert vorzugsweise von 4,0 bis 8,0, bevorzugter von 4,2 bis 7,5, noch bevorzugter von 4,4 bis 6,8, am Bevorzugtesten von 4,6 bis 6,0 oder 4,8 bis 5,5 und insbesondere von 5,0 bis 5,2 oder 5,0±0,1; gemessen bei Raumtemperatur (20 bis 23 °C). In einer bevorzugten Ausführungsform ist der pH-Wert 4,8 ± 1,0 oder 4,8 ± 0.8, bevorzugter 4,8 ± 0,7 oder 4,8 ± 0,6, noch bevorzugter 4,8 ± 0,5 oder 4,8 ± 0,4, am Bevorzugtesten 4,8 ± 0,3 oder 4,8 ± 0,2 und insbesondere 4,8 ± 0,1. In einer weiteren bevorzugten Ausführungsform ist der pH-Wert 5,0 ± 1,0 oder 5,0 ± 0.8, bevorzugter 5,0 ± 0,7 oder 5,0 ± 0,6, noch bevorzugter 5,0 ± 0,5 oder 5,0 ± 0,4, am Bevorzugtesten 5,0 ± 0,3 oder 5,0 ± 0,2 und insbesondere 5,0 ± 0,1. In einer weiteren bevorzugten Ausführungsform ist der pH-Wert 5,2 ± 1,0 oder 5,2 ± 0,8, bevorzugter 5,2 ± 0,7 oder 5,2 ± 0,6, noch bevorzugter 5,2 ± 0,5 oder 5,2 ± 0,4, am Bevorzugtesten 5,2 ± 0,3 oder 5,2 ± 0,2 und insbesondere 5,2 ± 0,1. In einer weiteren bevorzugten Ausführungsform ist der pH-Wert 5,5 ± 1,0 oder 5,5 ± 0,8, bevorzugter 5,5 ± 0,7 oder 5,5 ± 0,6, noch bevorzugter 5,5 ± 0,5 oder 5,5 ± 0,4, am Bevorzugtesten 5,5 ± 0,3 oder 5,5 ± 0,2 und insbesondere 5,5 ± 0,1. In einer weiteren bevorzugten Ausführungsform ist der pH-Wert 6,0 ± 1,0 oder 6,0 ± 0.8, bevorzugter 6,0 ± 0,7 oder 6,0 ± 0,6, noch bevorzugter 6,0 ± 0,5 oder 6,0 ± 0,4, am Bevorzugtesten 6,0 ± 0,3 oder 6,0 ± 0,2 und insbesondere 6,0 ± 0,1. In einer weiteren bevorzugten Ausführungsform ist der pH-Wert 6,5 ± 1,0 oder 6,5 ± 0.8, bevorzugter 6,5 ± 0,7 oder 6,5 ± 0,6, noch bevorzugter 6,5 ± 0,5 oder 6,5 ± 0,4, am Bevorzugtesten 6,5 ± 0,3 oder 6,5 ± 0,2 und insbesondere 6,5 ± 0,1. In einer weiteren bevorzugten Ausführungsform ist der pH-Wert 7,0 ± 1,0 oder 7,0 ± 0.8, bevorzugter 7,0 ± 0,7 oder 7,0 ± 0,6, noch bevorzugter 7,0 ± 0,5 oder 7,0 ± 0,4, am Bevorzugtesten 7,0 ± 0,3 oder 7,0 ± 0,2 und insbesondere 7,0 ± 0,1. In einer weiteren bevorzugten Ausführungsform ist der pH-Wert 7,4 ± 1,0 oder 7,4 ± 0.8, bevorzugter 7,4 ± 0,7 oder 7,4 ± 0,6, noch bevorzugter 7,4 ± 0,5 oder 7,4 ± 0,4, am Bevorzugtesten 7,4 ± 0,3 oder 7,4 ± 0,2 und insbesondere 7,4 ± 0,1. In einer weiteren bevorzugten Ausführungsform ist der pH-Wert 8,0 ± 1,0 oder 8,0 ± 0.8, bevorzugter 8,0 ± 0,7 oder 8,0 ± 0,6, noch bevorzugter 8,0 ± 0,5 oder 8,0 ± 0,4, am Bevorzugtesten 8,0 ± 0,3 oder 8,0 ± 0,2 und insbesondere 8,0 ± 0,1.

Die erfindungsgemäße Dialyselösung enthält ein oder mehrere (z.B. zwei, drei, vier oder fünf) erfindungsgemäße molekular geprägte Polymere; wobei diese wie oben stehend definiert sind.

Die erfindungsgemäße Dialyselösung enthält erfindungsgemäßes molekular geprägtes Polymer in einer Gesamtkonzentration von vorzugsweise 0,001 mM bis 10 M oder 0,01 bis 1,0 M, bevorzugter 0,10 bis 500 mM, noch bevorzugter 1,0 bis 250 mM, am Bevorzugtesten 10 bis 100 mM und insbesondere 25 bis 90 mM. In einer bevorzugten Ausführungsform ist die Gesamtkonzentration 25 ± 24 mM, bevorzugter 25 ± 20 mM, noch bevorzugter 25 ± 15 mM, am Bevorzugtesten 25 ± 10 mM und insbesondere 25 ± 5 mM. In einer weiteren bevorzugten Ausführungsform ist die Gesamtkonzentration 50 ± 25 mM, bevorzugter 50 ± 20 mM, noch bevorzugter 50 ± 15 mM, am Bevorzugtesten 50 ± 10 mM und insbesondere 50 ± 5 mM. In einer weiteren bevorzugten Ausführungsform ist die Gesamtkonzentration 75 ± 25 mM, bevorzugter 75 ± 20 mM, noch bevorzugter 75 ± 15 mM, am Bevorzugtesten 75 ± 10 mM und insbesondere 75 ± 5 mM. In einer weiteren bevorzugten Ausführungsform ist die Gesamtkonzentration 100 ± 25 mM, bevorzugter 100 ± 20 mM, noch bevorzugter 100 ± 15 mM, am Bevorzugtesten 100 ± 10 mM und insbesondere 100 ± 5 mM. In einer weiteren bevorzugten Ausführungsform ist die Gesamtkonzentration 200 ± 25 mM, bevorzugter 200 ± 20 mM, noch bevorzugter 200 ± 15 mM, am Bevorzugtesten 200 ± 10 mM und insbesondere 200 ± 5 mM. Die Gesamtkonzentration ist vorzugsweise berechnet mittels des mittleren Molekulargewichts der erfindungsgemäßen Polymere.

Die erfindungsgemäße Dialyselösung enthält erfindungsgemäßes molekular geprägtes Polymer in einer Gesamtmassenkonzentration von vorzugsweise 0,01 g/L bis 1,0 kg/L, bevorzugter von 0,1 bis 750 g/L, noch bevorzugter von 1,0 bis 500 g/L, am Bevorzugtesten 10 bis 250 g/L und insbesondere von 100 bis 200 g/L. In einer bevorzugten Ausführungsform ist die Gesamtmassenkonzentration 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

Die erfindungsgemäße Dialyselösung kann auch weitere osmotisch aktive Substanzen wie beispielsweise Glucose, Polyglucose, quervernetzte Glucose oder Polyglucose, Mannitol oder Glycerol enthalten.

Die erfindungsgemäße Dialyselösung enthält vorzugsweise einen oder mehrere Elektrolyte.

Im Sinne dieser Erfindung steht der Ausdruck "Elektrolyt" für eine Substanz, die freie Ionen enthält und elektrische Konduktivität aufweist. Vorzugsweise dissoziiert das Elektrolyt vollständig in Kationen und Anionen ohne den pH-Wert einer wässrigen Zusammensetzung im Wesentlichen zu ändern. Diese Eigenschaft grenzt Elektrolyte von Puffersubstanzen ab. Vorzugsweise liegen die Elektrolyte in einer Konzentration vor, die in einer im Wesentlichen vollständigen Dissoziation in Wasser resultiert.

Bevorzugte Elektrolyte sind ausgewählt aus der Gruppe der Alkalimetalle wie beispielsweise Na⁺ und K⁺ und der Erdalkalimetalle wie beispielsweise Ca²⁺ und Mg²⁺. Ein bevorzugtes Anion ist Cl⁻.

Die erfindungsgemäße Dialyselösung kann weitere Anionen wie beispielsweise Hydrogencarbonat, Dihydrogenphosphat, Hydrogenphosphat, Phosphat, Acetat, Lactat und Pyruvat enthalten; diese Anionen (in geeigneten Kombinationen mit Kationen) werden jedoch aufgrund Ihrer Pufferkapazität im Sinne dieser Erfindung nicht als Elektrolyte sondern als Puffer bezeichnet.

In einer bevorzugten Ausführungsform enthält die erfindungsgemäße Dialyselösung Na⁺-Ionen. Die Konzentration an Na⁺-Ionen ist vorzugsweise 10 bis 200 mM oder 50 bis 190 mM, bevorzugter 100 bis 180 mM oder 110 bis 170 mM, noch bevorzugter 115 bis 165 mM oder 120 bis 160 mM, am Bevorzugtesten 125 bis 155 mM und insbesondere 130 bis 150 mM. In einer weiteren bevorzugten Ausführungsform enthält die erfindungsgemäße Dialyselösung keine Na⁺-Ionen.

In einer bevorzugten Ausführungsform enthält die erfindungsgemäße Dialyselösung K⁺-Ionen. Die Konzentration an K⁺-Ionen ist vorzugsweise 0,10 bis 20 mM, bevorzugter 0,25 bis 15 mM, noch bevorzugter 0,50 bis 10 mM, am Bevorzugtesten 0,75 bis 7,5 mM und insbesondere 1,0 bis 5,0 mM. In einer weiteren bevorzugten Ausführungsform ist die Konzentration an K⁺-Ionen 1,0 ± 0,75, 2,0 ± 0,75, 3,0 ± 0,75, 4,0 ± 0,75 oder 5,0 ± 0,75 mM und insbesondere 1,0 ± 0,50, 2,0 ± 0,50, 3,0 ± 0,50, 4,0 ± 0,50 oder 5,0 ± 0,50. In einer weiteren bevorzugten Ausführungsform enthält die erfindungsgemäße Dialyselösung keine K⁺-Ionen.

In einer bevorzugten Ausführungsform enthält die erfindungsgemäße Dialyselösung Ca²⁺-Ionen. Die Konzentration an Ca²⁺-Ionen ist vorzugsweise 0,1 bis 3 mM, bevorzugter 0,25 bis 2,75 mM, noch bevorzugter 0,5 bis 2,5 mM, am Bevorzugtesten 0,75 bis 2,25 mM und insbesondere 1 bis 2 mM. In einer weiteren bevorzugten Ausführungsform ist die Konzentration an Ca²⁺-Ionen 0,25, 0,5, 0,75, 1, 1,25, 1,5, 1,75 oder 2 mM. In einer weiteren bevorzugten Ausführungsform enthält die erfindungsgemäße Dialyselösung keine Ca²⁺-Ionen.

In einer bevorzugten Ausführungsform enthält die erfindungsgemäße Dialyselösung Mg²⁺-Ionen. Die Konzentration an Mg²⁺-Ionen ist vorzugsweise 0,01 bis 1 mM, bevorzugter 0,05 bis 0,75 mM, noch bevorzugter 0,1 bis 0,5 mM, am Bevorzugtesten 0,15 bis 0,4 mM und insbesondere 0,2 bis 0,3 mM. In einer weiteren bevorzugten Ausführungsform ist die Konzentration an Mg²⁺-Ionen 0,05, 0,075, 0,1, 0,2, 0,25, 0,50 oder 0,75 mM. In einer weiteren bevorzugten Ausführungsform enthält die erfindungsgemäße Dialyselösung keine Mg²⁺-Ionen.

In einer bevorzugten Ausführungsform enthält die erfindungsgemäße Dialyselösung Cl⁻-Ionen. Die Konzentration an Cl⁻-Ionen ist vorzugsweise 10 bis 300 mM, bevorzugter 25 bis 250 mM, noch bevorzugter 50 bis 200 mM, am Bevorzugtesten 75 bis 150 mM und insbesondere 80 bis 125 mM. In einer weiteren bevorzugten Ausführungsform ist die Konzentration an Cl⁻-Ionen 100 ± 50 mM, bevorzugter 100 ± 25 mM, am Bevorzugtesten 100 ± 10 mM und insbesondere 96 ± 4 mM. In einer weiteren bevorzugten Ausführungsform enthält die erfindungsgemäße Dialyselösung keine Cl⁻-Ionen.

Die erfindungsgemäße Dialyselösung enthält vorzugsweise einen oder mehrere Puffer.

Geeignete Puffer sind dem Fachmann bekannt. Üblicherweise umfassen Puffer Lactat-, Hydrogencarbonat-, Carbonat-, Dihydrogenphosphat-, Hydrogenphosphat-, Phosphat-, Pyruvat-, Citrat-, Isocitrat-, Succinat-, Fumarat-, Acetat- und Lactat-Salze. Der Fachmann weiß, dass das entsprechende Kation der vorstehend genannten Anionen Bestandteil des Puffers ist, der zur Einstellung des pH-Wertes benutzt wird (z.B. Na^{⊕} als Bestandteil des Puffers NaHCO₃). Wenn das Puffersalz jedoch in Wasser dissoziiert hat es auch die Wirkung eines Elektrolyts. Für die Zwecke dieser Beschreibung berechnen sich die Konzentrationen an Kationen oder Anionen und die Gesamtkonzentration an Ionen, unabhängig davon, ob sie als Bestandteil von Elektrolyten, Puffern oder anderen Verbindungen (z.B. als Salz der erfindungsgemäßen Polymere) eingesetzt werden.

In einer bevorzugten Ausführungsform enthält der Puffer Hydrogencarbonat. Hydrogencarbonat ist ein gut verträgliches Puffersystem, das im alkalischen Milieu mit Carbonat und im sauren Milieu mit H₂CO₃ bzw. CO₂ im Gleichgewicht steht. Neben Hydrogencarbonat sind auch andere Puffer-Systeme einsetzbar, die im Bereich von pH 4 bis pH 8, bevorzugter im Bereich von pH 5 bis pH 7,6 und insbesondere im Bereich von pH 7,6, 7,4, 7,2 und/oder 7,0 eine Pufferwirkung ausüben; z.B. auch Verbindungen, die im Körper zu Hydrogencarbonat metabolisiert werden können wie Lactat oder Pyruvat.

In einer weiteren bevorzugten Ausführungsform enthält der Puffer das Salz einer schwachen Säure, vorzugsweise Lactat. Die Säurestärke (pKₛ) der schwachen Säure ist vorzugsweise ≤5. Der Puffer kann auch ein Gemisch von Substanzen mit Pufferwirkung sein, z.B. ein Gemisch enthaltend Hydrogencarbonat und ein Salz einer schwachen Säure (z.B. Lactat). Eine geringe Hydrogencarbonat-Konzentration hat den Vorteil, dass der CO₂-Druck im Behältnis gering ist.

In einer bevorzugten Ausführungsform ist die erfindungsgemäße Dialyselösung gepuffert durch Hydrogencarbonat. Die Hydrogencarbonat-Konzentration ist vorzugsweise 1,0 bis 200 mM, bevorzugter 2,5 bis 150 mM, noch bevorzugter 5 bis 100 mM, am Bevorzugtesten 5 bis 75 mM oder 10 bis 50 mM und insbesondere 20 bis 30 mM. In einer weiteren bevorzugten Ausführungsform ist die Hydrogencarbonat-Konzentration 25 mM. In einer weiteren bevorzugten Ausführungsform enthält die erfindungsgemäße Dialyselösung kein Hydrogencarbonat.

In einer bevorzugten Ausführungsform ist die erfindungsgemäße Dialyselösung gepuffert durch Lactat. Die Lactat-Konzentration ist vorzugsweise 1,0 bis 200 mM, bevorzugter 2,5 bis 150 mM, noch bevorzugter 5 bis 100 mM, am Bevorzugtesten 10 bis 50 mM oder 10 bis 25 mM und insbesondere 15 mM. In einer weiteren bevorzugten Ausführungsform enthält die erfindungsgemäße Dialyselösung kein Lactat.

In einer bevorzugten Ausführungsform ist die erfindungsgemäße Dialyselösung gepuffert durch Acetat. Die Acetat-Konzentration ist vorzugsweise 1,0 bis 100 mM, bevorzugter 1,0 bis 50 mM, noch bevorzugter 1,0 bis 25 mM, am Bevorzugtesten 1,0 bis 10 mM oder 2,0 bis 7,5 mM und insbesondere 2,5 bis 7,0 mM. In einer weiteren bevorzugten Ausführungsform enthält die erfindungsgemäße Dialyselösung kein Acetat.

Das Gesamtvolumen an Dialyselösung ist nicht beschränkt. Üblicherweise beträgt das Volumen mehrere Liter (geeignetes Verabreichungsvolumen für einen Patienten) bis einige hundert Liter (geeignetes Vorratsvolumen für mehr als einen Patienten).

Wie bereits oben stehend ausgeführt ist unter dem Ausdruck "Dialyselösung" im Sinne dieser Erfindung eine gebrauchsfertige Dialyselösung zu verstehen, d.h. die Dialyselösung kann direkt für die Dialysebehandlung (Hämodialyse oder Peritonealdialyse) verwendet werden.

In einer bevorzugten Ausführungsform ist die erfindungsgemäße Dialyselösung eine Peritonealdialyselösung wie nachstehend beschrieben.

Die Peritonealdialyselösung ist biochemisch so abgestimmt, dass sie die mit Nierenversagen einhergehende metabolische Azidose im Wesentlichen korrigiert. Die Peritonealdialyselösung enthält Hydrogencarbonat vorzugsweise in annähernd physiologischen Konzentrationen. In einer bevorzugten Ausführungsform enthält die Peritonealdialyselösung Hydrogencarbonat in einer Konzentration von ca. 20 bis 30 mM. In einer weiteren bevorzugten Ausführungsform enthält die Peritonealdialyselösung eine Hydrogencarbonat-Konzentration von 25 mM.

Ferner enthält die Peritonealdialyselösung vorzugsweise Kohlenstoffdioxid mit einem Partialdruck (pCO₂) von weniger als 60 mmHg. In einer bevorzugten Ausführungsform ist pCO₂ der Peritonealdialyselösung im Wesentlichen gleich zum pCO₂, der in Blutgefäßen gemessen wird.

Ferner hat die Peritonealdialyselösung vorzugsweise einen pH-Wert von ca. 7,4. Daher ist die Peritonealdialyselösung eine physiologisch verträgliche Lösung.

Die Peritonealdialyselösung enthält vorzugsweise eine schwache Säure mit einem pKₛ≤5. Die schwachen Säuren sind vorzugsweise Verbindungen, die als physiologische Stoffwechselprodukte im Glucose-Metabolismus auftreten. Die schwache Säure ist vorzugsweise ausgewählt aus Gruppe bestehend aus Lactat, Pyruvat, Citrat, Isocitrat, Ketoglutarat, Succinat, Fumarat, Malat und Oxaloacetat. Diese Säuren können entweder allein oder als Gemisch in der Peritonealdialyselösung enthalten sein. Die schwachen Säuren sind vorzugsweise in einer Konzentration von 10 bis 20 mEq/L und im Wesentlichen als NatriumSalze in der Peritonealdialyselösung enthalten. In der Peritonealdialyselösung ist die schwache Säure vorzugsweise in einer Menge enthalten, die der täglichen metabolischen Wasserstoffproduktion von ca. 1 mEq/kg/Tag entspricht.

Die Peritonealdialyselösung enthält wenigstens ein erfindungsgemäßes molekular geprägtes Polymer wie oben stehend definiert.

Die erfindungsgemäße Peritonealdialyselösung enthält vorzugsweise eine Konzentration an Hydrogencarbonat und weist einen pCO₂ auf, wie sie bei gesunden, nicht-niereninsuffizienten Patienten gemessen werden. Die schwache Säure diffundiert entlang des Konzentrationsgradienten von der Dialyselösung ins Blut des Dialysepatienten und korrigiert somit die metabolische Azidose der Dialysepatienten.

Ein weiterer Gegenstand dieser Erfindung betrifft Mehrkomponenten-Systeme zur Herstellung der oben beschriebenen gebrauchsfertigen Dialyselösungen. Die Herstellung erfolgt vorzugsweise in einer detailliert beschriebenen Art und Weise, d.h. durch Befolgung einer entsprechenden Anleitung (Protokoll). Besagte Herstellung kann manuell, z.B. durch Mischen einzelner Komponenten oder Verdünnen einer Komponente mit Wasser, erfolgen. Die Herstellung kann jedoch auch automatisiert erfolgen, z.B. mittels einer Vorrichtung die für diesen Prozess geeignet ist und kommerziell erhältlich sein kann. Die Herrichtung muss nicht zwangsläufig zu einer Dialyselösung mit statischer (gleich bleibender) Zusammensetzung führen sondern kann auch zu einer Dialyselösung führen, die kontinuierlich ihre Zusammensetzung ändert, wobei diese Änderung durch eine geeignete Vorrichtung überwacht werden kann. Beispielsweise kann das erfindungsgemäße molekular geprägte Polymer in einer Dialyselösung enthalten sein, die kontinuierlich während der Dialysebehandlung verdünnt wird, so dass der Patient einer abnehmenden Polymer-Konzentration ausgesetzt ist.

In einer bevorzugten Ausführungsform sind die erfindungsgemäßen Dialyselösungen zur Verwendung in der Behandlung der Niereninsuffizienz geeignet.

In einer weiteren bevorzugten Ausführungsform sind die erfindungsgemäßen Dialyselösungen zur Verwendung in der Dialysebehandlung geeignet.

In einer weiteren bevorzugten Ausführungsform sind die erfindungsgemäßen Dialyselösungen zur Verwendung in der Hämodialyse- und / oder Peritonealdialysebehandlung geeignet.

Das Mehrkammer-Behältnis kann als Mehrkammer-Kunststoffbeutel vorliegen, der jeweils eine abgetrennte Kammer für jede einzelne Komponente enthält. Vorzugsweise enthält das Behältnis die einzelnen Komponentenlösungen in Kammern, die jeweils durch Trennelemente voneinander abgetrennt sind.

Das Mehrkammer-Behältnis ist vorzugsweise ein Zweikammer-Beutel mit einer ersten Kammer und einer zweiten Kammer, wobei die Kammern durch ein lösbares bzw. brechbares Trennsystem voneinander getrennt sind, und die erste Kammer die erste Komponente enthält und die zweite Kammer die zweite Komponente enthält. Das Lösen bzw. Brechen des Trennsystems führt zum Mischen der beiden Komponenten und resultiert in der gebrauchsfertigen Dialyselösung. Die erste Kammer und die zweite Kammer sind im Behältnis vorzugsweise angrenzend angeordnet und durch das Trennsystem voneinander abgetrennt. Das Trennsystem ist vorzugsweise eine Trennnaht (z.B. lösbare oder brechbare Schweißnaht). Die Trennnaht öffnet sich vorzugsweise durch das Anlegen eines Druckes auf eine der Kammern, woraufhin die Trennnaht bricht bzw. sich löst und sich der Inhalt der beiden Kammern mischt und das Gemisch als gebrauchsfertige Dialyselösung in der Dialysebehandlung eingesetzt werden kann.

Der Fachmann erkennt, dass das Mischen der einzelnen Komponenten üblicherweise einen Verdünnungseffekt für den Fall nach sich zieht, dass die Komponenten die Inhaltsstoffe in unterschiedlichen Konzentrationen enthalten. Falls beispielsweise das erfindungsgemäße Polymer ausschließlich in einer der Komponenten enthalten ist, führt das Mischen dieser Komponente mit wenigstens einer anderen Komponente zu einem Anstieg des Volumens in Bezug auf die vorhandene Menge des erfindungsgemäßen Polymers und somit zu einer Verdünnung, d.h. Abnahme der Polymer-Konzentration; folglich enthält die Komponente das erfindungsgemäße Polymer vorzugsweise in einer höheren Konzentration als die gebrauchsfertige Dialyselösung.

Vorzugsweise ist die Konzentration an erfindungsgemäßem Polymer in der Komponente nahe an der Sättigungskonzentration bei einer Temperatur von 5 °C um eine ausreichende Lagerstabilität bei höheren Temperaturen sicher zu stellen.

In einer bevorzugten Ausführungsform ist die Gesamtmassenkonzentration an erfindungsgemäßen Polymer in der Komponente 0,01 g/L bis 1,0 kg/L, bevorzugter 0,1 bis 750 g/L, noch bevorzugter 1,0 bis 500 g/L, am Bevorzugtesten 10 bis 250 g/L und insbesondere 100 bis 200 g/L. In einer weiteren bevorzugten Ausführungsform ist die Gesamtmassenkonzentration an erfindungsgemäßen Polymer in der Komponente 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration an erfindungsgemäßen Polymer in der Komponente 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration an erfindungsgemäßen Polymer in der Komponente 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration an erfindungsgemäßen Polymer in der Komponente 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration an erfindungsgemäßen Polymer in der Komponente 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

In einer bevorzugten Ausführungsform enthält die zweite Komponente die Gesamtmenge an erfindungsgemäßen molekular geprägten Polymer und einen geeigneten Puffer, der den pH-Wert der zweiten Komponente auf über 7,0, bevorzugter auf über 7,5, noch bevorzugter auf über 8,0, am Bevorzugtesten auf über 8,5 und insbesondere auf über 9,0 einstellt. Dies kann vorzugsweise durch Hydrogencarbonat erreicht werden, die beispielsweise in Form von dissoziiertem Natrium-Hydrogencarbonat und oder Kalium-Hydrogencarbonat vorliegen können. In einer weiteren bevorzugten Ausführungsform ist die zweite Komponente fest und umfasst ein pulverförmiges Gemisch enthaltend wenigstens ein erfindungsgemäßes Polymer und wenigstens einen Puffer, z.B. Natrium- und/ oder Kalium-Hydrogencarbonat.

Der Mehrkammer-Beutel ist vorzugsweise geeignet für die Herstellung einer Dialyselösung, die zur Verwendung in der Peritonealdialysebehandlung verwendet werden kann, und die folgenden Inhaltsstoffe vorzugsweise in folgenden Konzentrationen enthält:

| | |
|---|---|
| Ca^{2⊕} | 0,5 bis 5 mval/L; |
| Mg^{2⊕} | 0 bis 3,0 mval/L; |
| Cl^{Θ} | 90,5 bis 121 mval/L; |
| K^{⊕} | 0 bis 4,0 mval/L; |
| HCO₃^{Θ} | 25 bis 40 mval/L; wobei |

eine Kammer des Mehrkammer-Beutel-Systems ein erstes saures Konzentrat und eine andere Kammer ein zweites basisches Konzentrat enthält; wobei das saure Konzentrat Ca^{2⊕}-Ionen enthält und das basische Konzentrat HCO₃^{Θ}-Ionen aber keine Ca^{2⊕}-Ionen enthält; und die zwei Konzentrate nach Lösen bzw. Brechen des Trennsystems (z.B. Trennnaht) miteinander gemischt werden können; wobei das Mischen der zwei Konzentrate zur Herstellung der gebrauchsfertigen Dialyselösung führt und der pH der gebrauchsfertigen Dialyselösung 7,0 bis 7,6 ist.

Vorzugsweise enthält das basische Konzentrat wenigstens ein erfindungsgemäßes Polymer und ggf. Glucose und/ oder Polyglucose, wohingegen das saure Konzentrat kein erfindungsgemäßes Polymer und keine Glucose und/ oder Polyglucose enthält.

Vorzugsweise enthält das basische Konzentrat eine Menge an Hydrogencarbonat, die zu einer Hydrogencarbonat-Konzentration der gebrauchsfertigen Dialyselösung von wenigstens 20 mM führt. Vorzugsweise ist die Hydrogencarbonat-Konzentration der basischen Komponente so hoch, dass die gebrauchsfertige Dialyselösung eine Hydrogencarbonat-Konzentration von 25 mM aufweist.

Der pH-Wert des basischen, gepufferten zweiten Konzentrats wird vorzugsweise mit Salzsäure eingestellt.

Vorzugsweise werden die beiden Konzentrate in einem Volumenverhältnis von 10:1 bis 1:10 oder 8:1 bis 1:8, bevorzugter 5:1 bis 1:5 oder 3:1 bis 1:3, noch bevorzugter 2:1 bis 1:2 und insbesondere 1:1 miteinander gemischt.

Der Mehrkammer-Beutel weist vorzugsweise eine Gasbarriere-Folie auf, die verhindert, das gasförmiges CO₂ aus dem System entweicht. Gasbarriere-Folien sind dem Fachmann bekannt.

Ein bevorzugter Gegenstand dieser Erfindung betrifft ein Verfahren zur Herstellung einer Dialyselösung, worin das gewünschte Mischverhältnis automatisch durch eine Dialysemaschine oder einen Peritonealdialyse-Cycler erfolgt.

Ein weiterer Gegenstand dieser Erfindung betrifft die Verwendung wenigstens eines erfindungsgemäßen Polymers zur Herstellung der erfindungsgemäßen Dialyselösung (Hämodialyselösung oder Peritonealdialyselösung).

### Beispiele

Die molekular geprägten Polymere wurden gemäß Hsieh et al., Biomaterials 27 (2006), 2083-2089 synthetisiert (Ziffer 2.2). Das Stoffmengenverhältnis von Monomer zu Vernetzungsmittel ist in allen Versuchen 1:10.

Die Bindungskapazität der synthetisierten Polymere wurde wie in Hsieh et al., Biomaterials, 27 (2006), 2083-2089 beschrieben bestimmt (Ziffer 2.4).

| Beispiel | Monomer | Mustermolekül¹⁾ | Imprinted Ratio |
|---|---|---|---|
| 1 | abgebaute Stärke²⁾ | --- | --- |
| 2 | abgebaute Stärke²⁾ | Kreatinin | >1,10 |
| 3 | abgebaute Stärke²⁾ | Kreatin | >1,10 |
| 4 | β-Cyclodextrin | --- | --- |
| 5 | β-Cyclodextrin | Kreatinin | >1,10 |
| 6 | β-Cyclodextrin | Hypoxanthin | >1,10 |
| 7 | Inulin³⁾ | --- | --- |
| 8 | Inulin³⁾ | Kreatinin | >1,10 |
| 9 | Inulin³⁾ | Indoxylsulfat | >1,10 |
| 10⁴⁾ | 4-Vpy und DVB⁵⁾ | --- | --- |
| 11⁴⁾ | 4-Vpy und DVB⁵⁾ | Kreatinin | >1,10 |

| | | | |
|---|---|---|---|
| ¹⁾ Mustermolekül ("Template") = urämisches Retentionssolut ¹⁾ Mittleres Molekulargewicht ∼ 6400 ³⁾ Mittleres Molekulargewicht ∼ 5000 ⁴⁾ Synthetisiert wie in Hsieh et al., Biomaterials 27 (2006), 2083-2089 (Ziffer 2.3) beschrieben ⁵⁾ 4-Vpy: 4-Vinylpyridin; DVB: Divinylbenzen | | | |

Die Eliminierung des Retentionssoluts Kreatinin wird wie folgt bestimmt:
Beispielverbindung 5 (3 g) wird in 10 mL einer 0,9% (m/m) wässriger NaCl-Lösung gelöst und in eine Kammer einer Pfeffer'schen Zelle eingebracht. Kreatinin (50 mg) wird in 10 mL einer 0,9% (m/m) wässriger NaCl-Lösung gelöst und in die andere Kammer der Pfeffer'schen Zelle eingebracht. Beide Zellen sind über eine semipermeable Membran (Cellulosemembran, Cut Off = 1000 Da) miteinander verbunden. Nach 16 Stunden werden die beiden Lösungen der Kammern mit Chloroform extrahiert (dreimalige Extraktion, 25 mL CHCl₃), das anschließend über Natriumsulfat getrocknet wird. Die getrocknete Chloroformphase wird im Rotationsverdampfer verdampft und der Rückstand wird anschließend für 24h im Vakuum getrocknet. Der Rückstand wird in 5 mL Wasser gelöst und die Kreatininkonzentration wird analytisch mittels HPLC (high performance liquid chromatography) bestimmt (Methode gemäß Hsieh et al., Biomaterials 27 (2006), 2083-2089 (Ziffer 2.6)).

Das Experiment wird mit Vergleichsbeispielverbindung 4 wiederholt.
Die Kammer mit Beispielverbindung 5 wies eine um ∼5% erhöhte Kreatininkonzentration imVergleich zur Kammer mit Vergleichsbeispielverbindung 4 auf.

## Patentansprüche

1. Molekular geprägtes Polymer, das durch wenigstens ein urämisches Retentionssolut geprägt ist, zur Verwendung in der Peritonealdialysebehandlung.

2. Molekular geprägtes Polymer zur Verwendung nach Anspruch 1, wobei das urämische Retentionssolut ausgewählt ist aus der Gruppe
1-Methyladenosin, 1-Methylguanosin, 1-Methylinosin, asymmetrisches Dimethylarginin, α-Keto-δ-guanidinovaleriansäure, α-N-Acetylarginin, Arab(in)itol, Argininsäure, Benzylalkohol, β-Guanidinopropionsäure, β-Lipotropin, Kreatin, Kreatinin, Cytidin, Dimethylglycin, Erythritol, γ-Guanidinobuttersäure, Guanidin, Guanidinoessigsäure, Guanidonobernsteinsäure, Hypoxanthin, Malondialdehyd, Mannitol, Methylguanidin, Myoinositol, N²,N²-Dimethylguanosin, N⁴-Acetylcytidin, N⁶-Methyladenosin, N⁶-Threonylcarbamoyladenosin, Orotsäure, Orotidin, Oxalat, Phenylacetylglutamin, Pseudouridin, symmterisches Dimethylarginin, Sorbitol, Taurocyamin, Threitol, Thymin, Uracil, Harnstoff, Harnsäure, Uridin, Xanthin, Xanthosin, 2-Methoxyresorcinol, 3-Deoxyglucoson, 3-Carboxy-4-methyl-5-propyl-2-furanproprionsäure, Fructoselysin, Glyoxal, Hippursäure, Homocystein, Hydroquinon, Indol-3-essigsäure, Indoxylsulfat, Kinurenin, Kynurensäure, Leptin, Melatonin, Methylglyoxal, N^{ε}-(carboxymethyl)lysin, p-Cresol, Pentosidin, Phenol, p-Hydroxyhippursäure, Putrescin, Quinolinsäure, Retinol-bindendes Protein, Spermidin, Spermin, Adrenomedullin, atriales natriuretisches Peptid, β₂-microglobulin, β-endorphin, Cholecystokinin, Clara Cell Protein (CC16), Komplementfaktor D, Cystatin C, Degranulation Inhibiting Protein, Delta-sleep Inducing Peptid, Endothelin, Hyaluronsäure, Interleukin-1β, Interleukin-6, κ-Ig Light Chain, λ-Ig Light Chain, Leptin, Methionin-Enkephalin, Neuropeptid Y, Parathyroidhormon, Retinol-bindendes Protein, Tumornekrosefaktor-α, 1-alkyl-2-formyl-3,4-glycosyl-pyrrol, 2-(2-Fuoryl)-4(5)-(2-furanyl)-1H-imidazole, 3-Deoxyfructoson, 3-Hydroxykinurenin, 4-Hydroxynonenal, AOPP (Advanced Oxidation Protein Products), Advanced Glycation End Products-β₂-Microglobulin, Anthranilsäure, β₂-Microglobulinfragmente, Cadaverin, Crossline, Dimethylamin, Guanosin, Imidazolon, Malonaldehyd, Malondialdehyd, Methylamin, N^{ε}-carboxyethyllysine, Organische Chloramine, oxidiertes Low-Density-Lipoprotein (oxLDL), Parathyroidhormonfragmente, Pyrralin, Pyrrolaldehyd, und Trimethylamin.

3. Molekular geprägtes Polymer zur Verwendung nach einem der Ansprüche 1 oder 2, wobei eine 7,5 gewichtsprozentigen wässrige Lösung des molekular geprägten Polymers einen kolloidosmotischen Druck ≥50 mosmol/L aufweist.

4. Molekular geprägtes Polymer zur Verwendung nach einem oder mehreren der Ansprüche 1 bis 3, wobei für das molekular geprägte Polymer von a) wenigstens einem Monomer abgeleitet ist oder b) von wenigstens einem Monomer und wenigstens einem Vernetzungsmittel abgeleitet ist

5. Molekular geprägtes Polymer zur Verwendung nach Anspruch 4, wobei das Monomer ein Saccharid, eine Aminosäure, Peptid oder ein olefinisch ungesättigtes Monomer ist.

6. Molekular geprägtes Polymer zur Verwendung nach Anspruch 5, wobei das Saccharid ausgewählt ist aus der Gruppe Glucose, Fructose, Arabinose, Xylose, Galactose, Mannose, N-Acetylglucosamin, Glucosamin, Stärke, abgebaute Stärke, Inulin, α-Cyclodextrin, β-Cyclodextrin und γ-Cyclodextrin.

7. Molekular geprägtes Polymer zur Verwendung nach Anspruch 4, wobei das Vernetzungsmittel ausgewählt ist aus der Gruppe Glyoxal, 1,2-Dihalogenethan, 1,3-Dihalogenpropan, Halogencarbonsäurehalogenid, Epichlorhydrin, 4-Chlor-1,2-epoxybutan, 1,2,3,4-Diepoxybutan, Tetramethylendiisocyanat und Hexamethylendiisocyanat

8. Peritonealdialyselösung enthaltend wenigstens ein molekular geprägtes Polymer nach einem oder mehreren der Ansprüche 1 bis 7.

9. Verwendung
- wenigstens eines molekular geprägten Polymers nach einem oder mehreren der Ansprüche 1 bis 7,
zur Herstellung der Peritonealdialyselösung nach Anspruch 8.

## Claims

1. A molecularly imprinted polymer, imprinted by at least one uremic retention solute, for use in peritoneal dialysis treatment.

2. The molecularly imprinted polymer, for use according to claim 1, wherein the uremic retention solute is selected from the group consisting of:
1-methyladenosine, 1-methylguanosiine, 1-methylinosine, asymmetric dimethylarginine, α-keto-δ-guanidinovaleric acid, α-N-acetylarginine, arab(in)itol, arginic acid, benzyl alcohol, β-guanidinopropionic acid, β-lipotropin, creatine, creatinine, cytidine, dimethylglycine, erythritol, γ-guanidinobutyric acid, guanidine, guanidineacetic acid, guanidonosuccinic acid, hypoxanthine, malondialdehyde, mannitol, methylguanidine, myoinositol, N²,N²-dimethylguanosine, N⁴-acetylcytidine, N⁶-methyladenosine, N⁶-threonylcarbamoyladenosine, orotic acid, orotidine, oxalate, phenylacetylglutamine, psuedouridine, symmetrical dimethylarginine, sorbitol, taurocyamine, threitol, thymine, uracil, urea, uric acid, uridine, xanthine, xanthosine, 2-methoxyresorcinol, 3-deoxyglucosone, 3-carboxy-4-methyl-5-propyl-2-furanpropionic acid, fructose lysine, glyoxal, hippuric acid, homocysteine, hydroquinone, indole-3-acetic acid, indoxyl sulfate, kynurenine, kynurenic acid, leptin, melatonin, methylglyoxal, N^{ε}-(carboxymethyl)lysine, p-cresol, pentosidine, phenol, p-hydroxyhippuric acid, putrescine, quinolinic acid, retinol-binding protein, spermidine, spermine, adrenomedullin, atrial natriuretic peptide, β²-microglobulin, β-endorphin, cholecystokinin, Clara cell protein (CC16), complement factor D, cystatin C, degranulation inhibiting protein, delta sleep-inducing peptide, endothelin, hyaluronic acid, interleukin-1β, interleukin-6, κ-lg light chain, λ-lg light chain, leptin, methionine enkephalin, neuropeptide Y, parathyroid hormone, retinol-binding protein, tumor necrosis factor α, 1-alkyl-2-formyl-3,4-glycosylpyrrole, 2-(2fuoryl)-4(5)-(2-furanyl)-1H-imidazole, 3-deoxyfructosone, 3-hydroxykynurenine, 4-hydroxynonenal, AOPP (advanced oxidation protein products), advanced glycation end products - β₂-microglobulin, anthranilic acid, β₂-microglobulin fragments, cadaverine, crossline, dimethylamine, guanosine, imidazolone, malonaldehyde, malondialdehyde, methylamine, N^{ε}-(carboxyethyl)lysine, organic chloramine, oxidized low-density lipoprotein (oxLDL), parathyroid hormone fragments, pyrraline, pyrrolaldehyde and trimethylamine.

3. The molecularly imprinted polymer for use according to any one of claims 1 or 2, wherein a 7.5% by weight aqueous solution of the molecularly imprinted polymer has a colloid osmotic pressure ≥50 mosmol/L.

4. The molecularly imprinted polymer for use according to any one of claims 1 to 3, wherein the molecularly imprinted polymer is derived from a) at least one monomer, or b) is derived from at least one monomer and at least one cross-linking agent.

5. The molecularly imprinted polymer for use according to claim 4, wherein the monomer is a saccharide, an amino acid, a peptide or an olefinically unsaturated monomer.

6. The molecularly imprinted polymer for use according to claim 5, wherein the saccharide is selected from the group consisting of glucose, fructose, arabinose, xylose, galactose, mannose, N-acetylglucosamine, glucosamine, starch, degraded starch, inulin, α-cyclodextrin, β-cyclodextrin and γ-cyclodextrin.

7. The molecularly imprinted polymer for use according to claim 4, wherein the cross-linking agent is selected from the group consisting of glyoxal, 1,2-dihaloethane, 1,3-dihalopropane, halocarboxylic acid halide, epichlorohydrin, 4-chloro-1,2-epoxybutane, 1,2,3,4-diepoxybutane, tetramethylene diisocyanate and hexamethylene diisocyanate.

8. A peritoneal dialysis solution containing at least one molecularly imprinted polymer according to any one or more of claims 1 to 7.

9. Use of
- at least one molecularly imprinted polymer according to any one or more of claims 1 to 7,
to prepare the peritoneal dialysis solution according to claim 8.

## Revendications

1. Polymère à empreinte moléculaire qui porte l'empreinte d'au moins un soluté de rétention urémique et destiné à une utilisation dans un traitement par dialyse péritonéale.

2. Polymère à empreinte moléculaire destiné à être utilisé selon la revendication 1, dans lequel le soluté de rétention est sélectionné dans le groupe suivant :
1-méthyladénosine, 1-méthylguanosine, 1-méthylinosine, diméthylarginine asymétrique, acide α-céto-δ-guanidinovalérianique, α-N-acétylarginine, arab(ine)itol, acide argininique, alcool benzylique, acide β-guanidinopropionique, β-lipotropine, créatine, créatinine, cytidine, diméthylglycine, érythritol, acide γ-guanidinobutyrique, guanidine, acide guanidinoacétique, acide guanidonosuccinique, gypoxanthine, malondialdéhyde, mannitol, méthylguanidine, myoinositol, N², N²-diméthylguanosine, N⁴-acétylcytidine, N⁶-méthyladenosine, N⁶-thréonylcarbamoyladénosine, acide orotique, orotidine, oxalate, phénylacétylglutamine, pseudouridine, diméthylarginine symétrique, sorbitol, taurocyamine, thréitol, thymine, uracil, urée, acide urique, uridine, xanthine, xanthosine, 2-méthoxyrésorcinol, 3-désoxyglucosone, acide 3-carboxy-4-méthyle-5-propyle-2-furanproprionique, lysine de fructose, glyoxal, acide hippurique, homocystéine, hydroquinone, acide indol-3-acétique, indoxylsulfate, kynurénine, acide kynurénique, leptine, mélatonine, méthylglyoxal, NE-(carboxyméthyl)lysine, p-crésol, pentosidine, acide phénol, p-hydroxyhippurique, putrescine, acide quinolinique, protéine liant le rétinol, spermidine, spermine, adrénomédulline, peptide atrial natriurétique, β₂-microglobuline, β-endorphine, cholécystokinine, protéine des cellules de Clara (CC16), facteurs complémentaires D, cystatine C, protéine inhibant la dégranulation, peptide induisant le sommeil profond, endothéline, acide hyaluronique, interleukine-1β, interleukine-6, chaîne légère de κ-Ig, chaîne légère de λ-Ig, leptine, méthionine enképhaline, neuropeptide Y, hormone parathyroïdienne, protéine liant le rétinol, facteur de nécrose tumorale-α, 1-alkyle-2-formyle-3,4-glycosyl-pyrrol, 2-(2-fuoryle)-4(5)-(2-furanyl)-1H-imidazole, 3-désoxy-fructosone, 3-hydroxykinurénine, 4-hydroxynonénal, AOPP (produits protéiques d'oxydation avancée), produits terminaux de glycation avancée-β₂-microglobuline, acide anthranilique, β₂-fragments de microglobuline, cadavérine, Crossline, diméthylamine, guanosine, imidazolone,malonaldéhyde, malondialdéhyde, méthylamine, N^{ε}-carboxyéthyllysine, chloramine organique, lipoprotéine à basse densité oxydée (oxLDL), fragments d'hormone parathyroïdienne, pyrraline, pyrrolaldéhyde, et triméthylamine.

3. Polymère à empreinte moléculaire destiné à être utilisé selon une des revendications 1 ou 2, dans lequel une solution aqueuse de 7,5 % en poids du polymère à empreinte moléculaire présente une pression colloïdale osmotique > 50 milliosmoles/l.

4. Polymère à empreinte moléculaire destiné à être utilisé selon une ou plusieurs des revendications 1 à 3, dans lequel pour le polymère à empreinte moléculaire, au moins un monomère est dérivé de a) ou b) est dérivé d'au moins un monomère et d'au moins un agent de réticulation.

5. Polymère à empreinte moléculaire destiné à être utilisé selon la revendication 4, dans lequel le monomère est un saccharide, un acide aminé, un peptide ou un monomère insaturé d'oléfine.

6. Polymère à empreinte moléculaire destiné à être utilisé selon la revendication 5, dans lequel le saccharide est sélectionné dans le groupe suivant : glucose, fructose, arabinose, xylose, galactose, mannose, N-acétylglucosamine, glucosamine, amidon, amidon décomposé, inuline, α-cyclodextrine, β-cyclodextrine et γ-cyclodextrine.

7. Polymère à empreinte moléculaire destiné à être utilisé selon la revendication 4, dans lequel l'agent de réticulation est sélectionné dans le groupe suivant : glyoxal, 1,2-dihalogène-éthane, 1,3-dihalogène-propane, halogénure d'acide halogénocarboxylique, épichlorhydrine, 4chloro-1,2-époxybutane, 1,2,3,4-diépoxybutane, tétraméthylène diisocyanate et hexaméthylène diisocyanate.

8. Solution de dialyse péritonéale contenant au moins un polymère à empreinte moléculaire selon une ou plusieurs des revendications 1 à 7.

9. Utilisation
- d'au moins un polymère à empreinte moléculaire selon une ou plusieurs des revendications 1 à 7
pour la préparation d'une solution de dialyse péritonéale selon la revendication 8.
